(19) Europäisches Patentamt
European Patent Office
Office européen des brevets

(11) Veröffentlichungsnummer: **0 621 277 A2**

(12) # EUROPÄISCHE PATENTANMELDUNG

(21) Anmeldenummer: **94105448.8**

(51) Int. Cl.[5]: **C07D 521/00**, A61K 31/33

(22) Anmeldetag: **08.04.94**

(30) Priorität: **23.04.93 DE 4313267**

(43) Veröffentlichungstag der Anmeldung:
**26.10.94 Patentblatt 94/43**

(84) Benannte Vertragsstaaten:
**AT BE CH DE DK ES FR GB GR IE IT LI NL SE**

(71) Anmelder: **BASF Aktiengesellschaft**
**Carl-Bosch-Strasse 38**
**D-67063 Ludwigshafen (DE)**

(72) Erfinder: **Wingert, Horst, Dr.**
**D 3.1**
**68159 Mannheim (DE)**
Erfinder: **Hellendahl, Beate, Dr.**
**Sebastian-Kneipp-Strasse 17**
**67105 Schifferstadt (DE)**
Erfinder: **Sauter, Hubert, Dr.**
**Neckarpromenade 20**
**68167 Mannheim (DE)**
Erfinder: **Lorenz, Gisela, Dr.**
**Erlenweg 13**
**67434 Neustadt (DE)**

(54) **Diarylderivate und deren Verwendung als Pflanzenschutzmittel.**

(57) Diarylderivate der allgemeinen Formel I

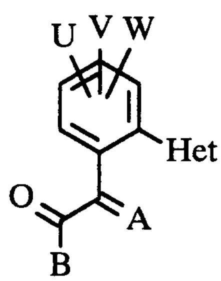

I

in der
A
$=CHOR^1$, $=CHSR^1$, $=CHR^1$, $=CHCl$ oder $=NOR^1$ bedeutet,
B
$OR^2$, $SR^2$ oder $NR^2R^3$ bedeutet
und
Het
für einen ein-, zwei- oder dreikernigen aromatischen Fünfring- oder Sechsringheterocyclus steht, der gegebenenfalls durch R substituiert sein kann,
$R^1$, $R^2$, $R^3$, $R^4$, $R^5$
gleich oder verschieden sind und Wasserstoff oder Alkyl bedeuten und
U, V, W
gleich oder verschieden sind und Halogen, Alkyl oder Alkoxy bedeuten und diese Verbindungen enthaltende Fungizide.

EP 0 621 277 A2

Die vorliegende Erfindung betrifft neue Diarylderivate der allgemeinen Formel I

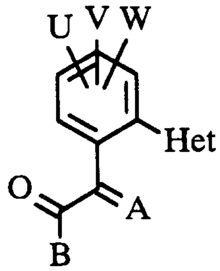

I

in der die Substituenten die folgende Bedeutung haben:

A

$=CHOR^1$, $=CHSR^1$, $=CHR^1$, $=CHCl$ oder $=NOR^1$,

B

$OR^2$, $SR^2$ oder $NR^2R^3$,

Het

ein ein-, zwei- oder dreikerniger aromatischer Fünfring- oder Sechsringheterocyclus, der gegebenenfalls einen bis drei der Reste R tragen kann,

R

unabhangig voneinander Halogen, Nitro, Cyano, $CO_2R^4$, $NR^4R^5$, $CONR^4R^5$, $S(O)_nR^4$ $P(O)$ $(OR^4)_2$, ggf. subst. $C_1$-$C_6$-Alkyl, $C_1$-$C_4$-Halogenalkyl, ggf. subst. $C_3$-$C_6$-Cycloalkyl, $C_1$-$C_4$Alkoxy-$C_1$-$C_4$-alkyl, $C_1$-$C_4$-Alkylthio-$C_1$-$C_4$-alkyl, ggf. subst. $C_2$-$C_6$-Alkenyl, ggf. subst. $C_2$-$C_6$-Alkinyl, $C_1$-$C_6$-Alkoxy, $C_1$-$C_4$-Thioalkoxy, ggf. subst. Benzylthio,
$C_1$-$C_4$-Alkylcarbonyl, ggf. subst. Phenylcarbonyl, ggf. subst. Aryl, ggf. subst. Aryloxy, ggf. subst. Arylthio, ggf. subst. Aryl-$C_1$-$C_4$-alkyl, ggf - subst. Aryl-$C_2$-$C_4$-alkenyl, ggf. subst. Aryl-$C_2$-$C_4$-alkinyl, ggf. subst. Aryloxy-$C_1$-$C_4$-alkyl, ggf. subst. Arylthio-$C_1$-$C_4$-alkyl, ggf. subst. Heteroaryl, ggf. subst. Heteroaryloxy, ggf. subst. Heteroaryl-$C_1$-$C_4$-alkyl, ggf. subst. Heteroaryl-$C_2$-$C_4$-alkenyl, ggf. subst. Heteroaryloxy-$C_1$-$C_4$-alkyl bedeuten, wobei "ggf. subst." für die Reste Halogen, Cyano, Nitro, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkoximino-$C_1$-$C_2$-alkyl steht und

wobei

n für 0, 1 oder 2 steht,

$R^1$, $R^2$, $R^3$, $R^4$, $R^5$

unabhängig voneinander Wasserstoff oder $C_1$-$C_4$-Alkyl und

U, V, W

unabhängig voneinander Halogen, $C_1$-$C_4$-Alkyl oder $C_1$-$C_4$-Alkoxy, Verfahren zur Bekämpfung von Schädlingen, insbesondere von Pilzen, Insekten, Nematoden und Spinnmilben mit diesen Verbindungen.

Es ist bekannt, 2-Phenyl-3-methoxyacrylsäuremethylester oder Phenylglyoxylsäuremethylester-O-methyloxime, die im Phenylrest in 2-Stellung durch Heteroarylmethyl, Heteroarylethenyl, Heteroaryloxy, Phenyl oder Heteroaryloxymethyl substituiert sind, als Fungizide zu verwenden (EP-A 178 826, EP-A 350 691, EP-A 363 818, EP-A 378 755, EP-A 254 426). Ihre fungizide Wirkung ist jedoch unbefriedigend.

Der Erfindung lagen neue Verbindungen mit verbesserten Eigenschaften zugrunde.

Demgemäß wurden die neuen Verbindungen I gefunden. Außerdem wurde gefunden, daß die Verbindungen I eine ausgezeichnete fungizide, insektizide, nematizide und akarizide Wirkung besitzen. Die fungizide und insektizide Wirkung wird bevorzugt.

Die Herstellung der Verbindungen der allgemeinen Formel I wird im Folgenden beschrieben:

Als Abkürzung wird für die Gruppierung

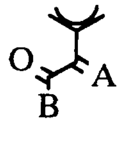

das Symbol

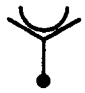

verwendet.

Die neuen Verbindungen der allgemeinen Struktur Ib lassen sich herstellen, indem man z.B. einen Bromaromaten der allgemeinen Formel 1 mit einer heteroaromatischen Zinnverbindung 2 bzw. mit einer heteroaromatischen Borverbindung 3 in Gegenwart eines Übergangsmetallkatalysators (ÜM), vorzugsweise in Gegenwart einer Nickel-, bzw. einer Palladium-Verbindung, wie beispielsweise $NiCl_2$, $Pd(OAc)_2$, $PdCl_2$ oder $Pd(PPh_3)_4$ in an sich bekannter Weise umsetzt (vgl. z.B. Synthesis, 693 (1987); T.R. Bailey, Tetrah. Lett. 4407 (1986); Y. Yamamoto, Heterocycles 16,1161 (1981)). Die heteroaromatischen Zinn- bzw. Borverbindungen sind bekannt, oder lassen sich analog zu bekannten Methoden herstellen.

Schema 1

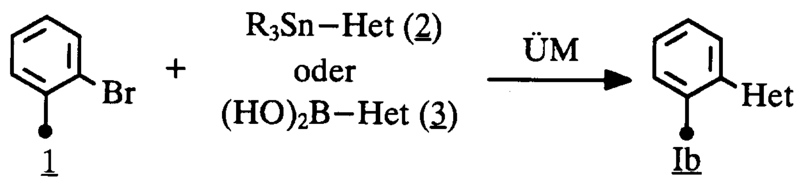


Als weiterer Weg zur Darstellung der Verbindungen der allgemeinen Formel I, in der der Ausdruck Het für einen aromatischen Fünfringheterocyclus steht bietet sich die [3 + 2]-dipolare Cycloaddition an.

So lassen sich die Acetylenderivate 4 in an sich bekannter Weise mit 1,3-Dipolen 5 zu den gewünschten aromatischen Fünfringheterocyclen 6 umsetzen (vgl. Houben-Weyl Bd. 5/2a, S. 830 ff (1977)).

Schema 2

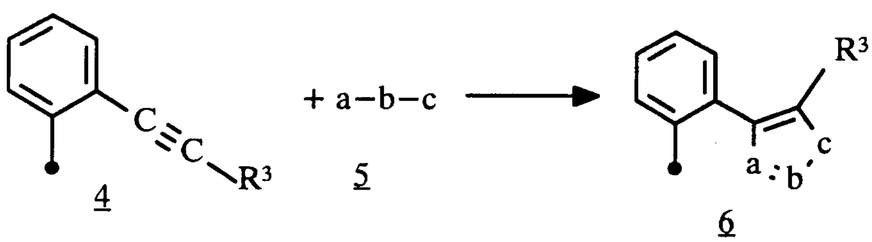

Auf diese Weise lassen sich beispielsweise die folgenden Derivate darstellen:

- durch Umsetzung mit Nitriloxiden 7 erhält man die Isoxazolderivate 8 bzw. 9,

Schema 3

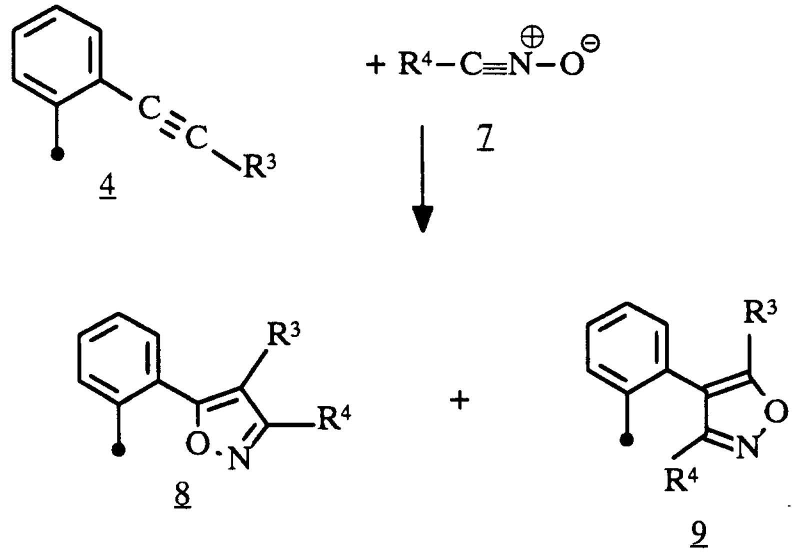

4 7 8 9

- Man kann auch so vorgehen, daß man ein Oxim der allgemeinen Formel 10 mit einem Acetylen 11 in Gegenwart von Hypochlorid zur Reaktion bringt. Man erhält dabei die Isoxazole 12 bzw. 13

Schema 4

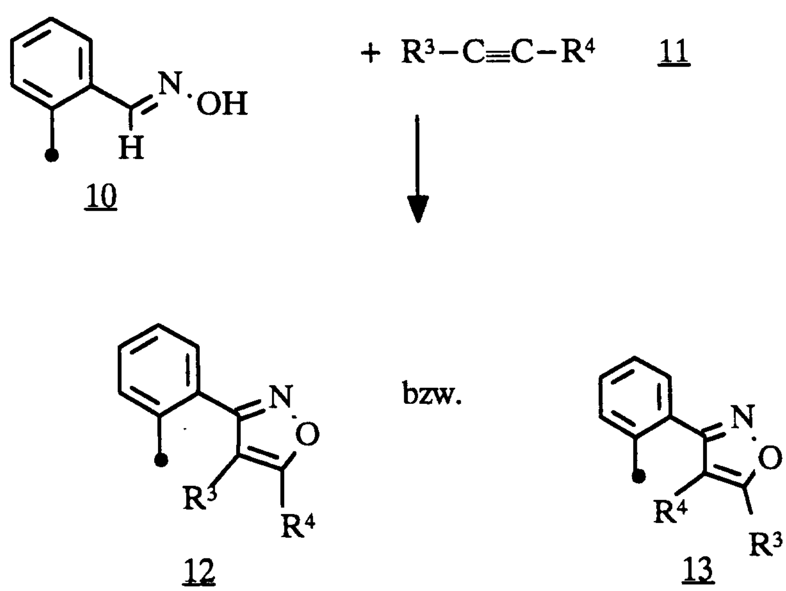

10 11 12 bzw. 13

- durch Umsetzung der Acetylene 4 mit Aziden 14 lassen sich die Triazole der allgemeinen Formel 15 darstellen:

Schema 5

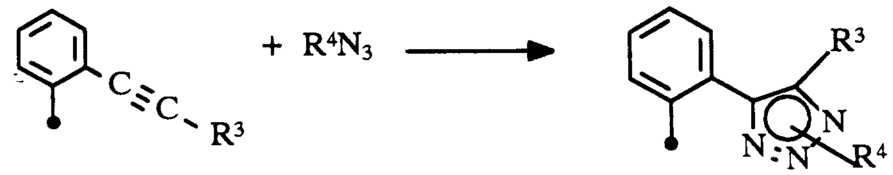

4 14 15

- durch Umsetzung mit Diazoalkanen 16 lassen sich die Pyrrole 17 bzw. 18 erhalten.

Schema 6

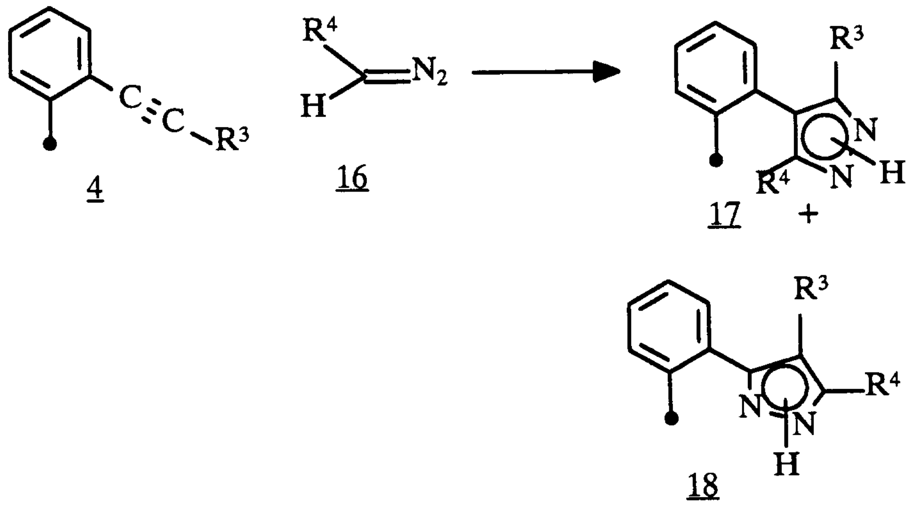

4 16 17 + 18

Oxadiazol-Derivate 22 lassen sich beispielsweise aus den Acylhydrazonen 21 darstellen, die ihrerseits aus den Aldehyden 19 durch Umsetzung mit Hydraziden 20 erhalten werden (vgl. EP 499823).

Schema 7

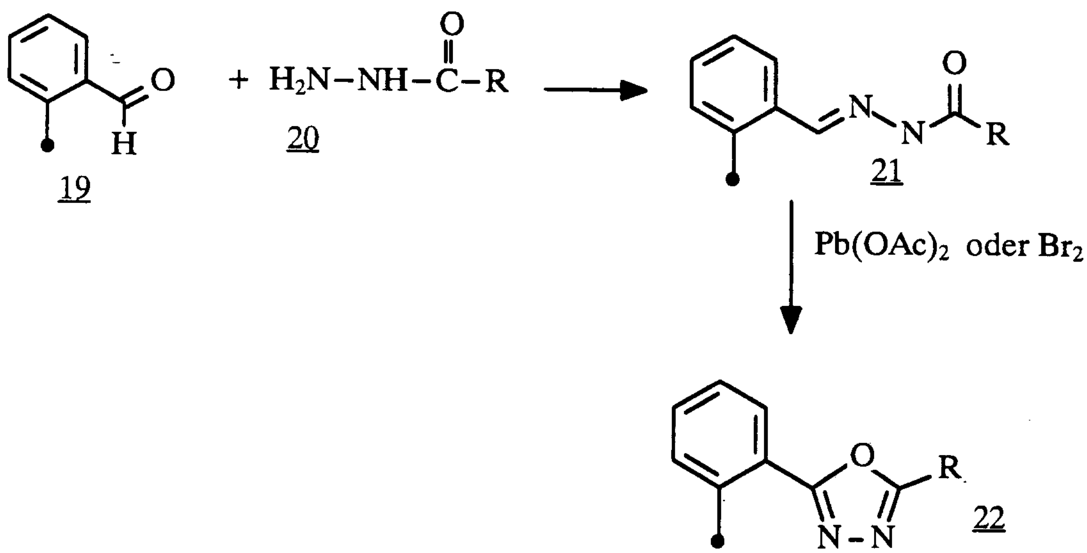


19 20 21 22

- Die Oxidation der Hydrazone 21 zu den Oxadiazolen 22 fuhrt man beispielsweise mit Bleitetraacetat oder mit Brom in einem Lösungs-oder Verdünnungsmittel wie etwa Methylenchlorid oder Chloroform durch (vgl. z.B. Synthesis, 411, 1986).

Eine weitere Moglichkeit zur Darstellung der neuen Verbindungen der Formel I ist in Schema 8 aufgezeigt:

Schema 8

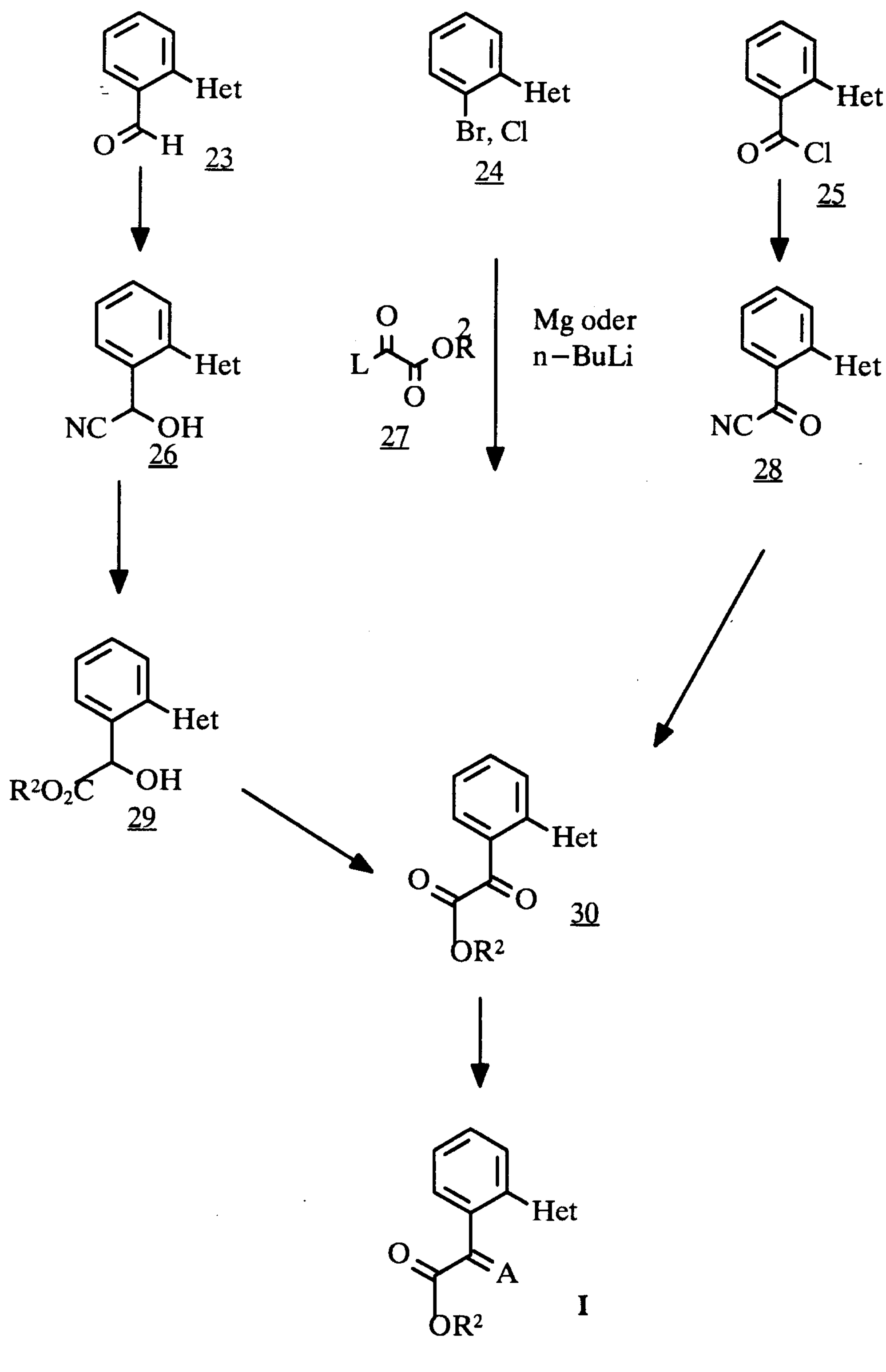


23 24 25 26 27 28 29 30 I

- Die Aldehyde lassen sich in an sich bekannter Weise über die Stufe der Cyanhydrine 26 in die Mandelsäurederivate 29 überführen (vgl. z.B. US-A 2892 847) diese können dann analog zu bekannten Methoden, beispielsweise mit Natriumhypochlorid zu den Ketoestern 30 oxidiert werden (vgl. z.B. EP-A 140 454).
- Die Ketoester 30 lassen sich auch in einer Stufe aus den Halogenderivaten 24 uber Grignard- bzw. Metallorganyl-Zwischenstufen darstellen (vgl. z.B. Synth. Comm. 20, 1781 (1990).

- Eine weitere Möglichkeit zur Darstellung der Ketoester bietet die Überführung der Säurechloride 25 in die Benzoylcyanide 28, die dann in einer Pinner-Reaktion in die Ketoester 30 überführt werden können (vgl. z.B. EP-A 493 711).

Aus den so dargestellten Ketoestern 30 lassen sich die Ester der allgemeinen Formel I erhalten, indem man

Schema 9

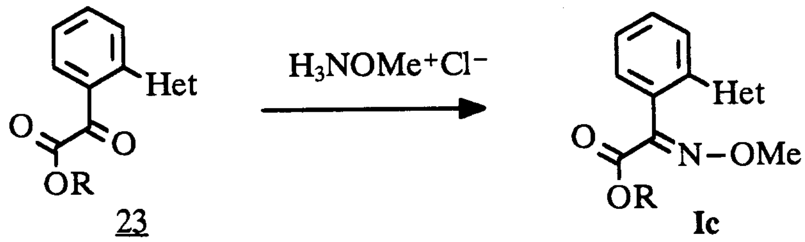


23 Ic

a) die Ketoester 23 mit Methoxiaminhydrochlorid in die Oximether Ic überführt, oder

Schema 10

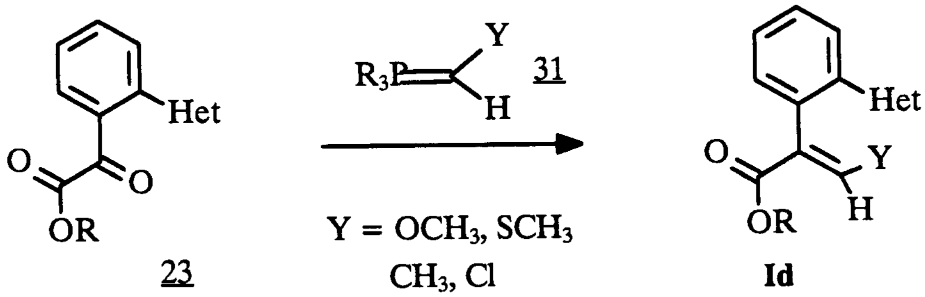


23 31 Id

b) die Ketoester 23 im Sinne einer Wittig-, bzw. einer Wittig-Horner-Reaktion, mit den Yliden 31 umsetzt.

Aus den so dargestellten Estern Ie lassen sich die Thiolester bzw. die Methylamide in an sich bekannter Weise auf folgendem Weg erhalten:

Schema 11

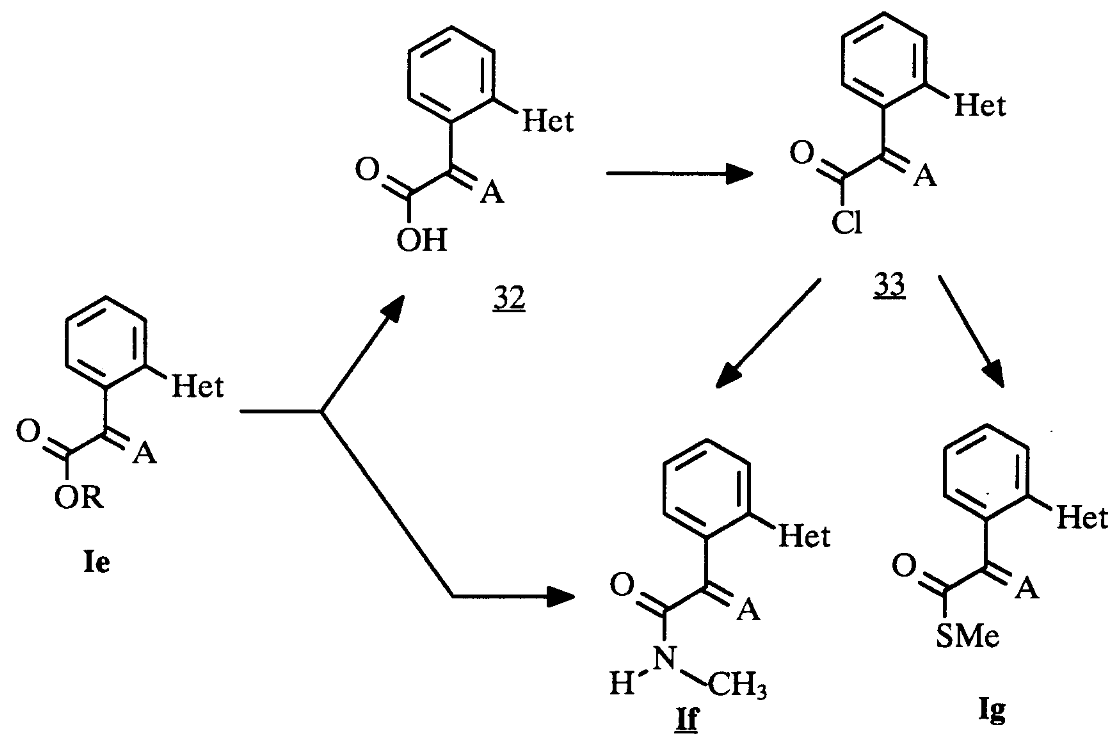

32 33 Ie If Ig

Die Thiolester Ig können über die Zwischenverbindungen Säure 32, Säurechlorid 33 in an sich bekannter Weise erhalten werden (vgl. EP-A 432 503).

Die Methylamide If lassen sich entweder direkt durch Aminolyse der Ester Ie darstellen (vgl. EP-A 477 631) oder auch durch Aminolyse der Säurechloride 33 (EP-A 477 631).

Die neuen Verbindungen der allgemeinen Formel I können bei der Herstellung aufgrund der C=C- bzw. C=N-Doppelbindungen als E/Z-Isomerengemisch anfallen. Diese können in der üblichen Weise, z.B. durch Kristallisation oder Chromatographie in die einzelnen Komponenten aufgetrennt werden. Sowohl die einzelnen isomeren Verbindungen als auch ihre Gemische werden von der Erfindung umfaßt und sind als Schädlingsbekämpfungsmittel brauchbar.

Die in der allgemeinen Formel I aufgeführten Reste können beispielsweise die folgende Bedeutung haben:

A

$=CHOR^1$, $=CHSR^1$, $=CHR^1$, $=CHCl$ oder $=NOR^1$,

B

$OR^2$, $SR^2$ oder $NR^2R^3$ und der Ausdruck

Het

ein ein-, zwei- oder dreikerniger aromatischer Fünfring-oder Sechsringheterocyclus, wie z.B. Pyridyl, Pyridazinyl, Pyrimidinyl, Pyrazinyl, Triazinyl, Tetrazinyl, Chinolinyl, Phenazinyl, Thienyl, Furyl, Pyrryl, Imidazolyl, Oxazolyl, Isoxazolyl, Thiazolyl, Thiadiazolyl, Oxadiazolyl, Tetrazolyl, Benzothienyl, Benzofuranyl, Indolyl, Benzimidazolyl, Benzooxazolyl, Benzothiazolyl, Benzisoxazolyl, der gegebenenfalls durch einen bis drei der Reste R substituiert sein kann, wobei die Reste

R

unabhängig voneinander Wasserstoff, Halogen (z.B. Fluor, Chlor, Brom, Jod), Nitro, Cyano, $NR^4R^5$, $CO_2R^4$, $CONR^4,R^5$, $S(O)_nR^4$ mit $n=0,1$ und 2, $P(O)(OR^4)_2$, ggf. verzweigtes $C_1$-$C_{10}$-Alkyl (z.B. Methyl, Ethyl, n-, iso-Propyl, n-, iso-, sec.-, tert.-Butyl, n-Pentyl, neo-Pentyl, n-Hexyl, n-Decyl), $C_1$-$C_4$-Halogenalkvl (z.B. Chlormethyl, Brommethyl, Fluormethyl, Dichlormethyl, Difluormethyl, Trifluormethyl, 1,2-Dibromethyl, 1,1,2,2-Tetrafluorethyl, Pentafluorethyl), $C_3$-$C_6$-Cycloalkyl (Cyclopropyl, 1-Methylcyclopropyl, Cyclobutyl, Cyclopentyl, Cyclohexyl), $C_1$-$C_4$-Alkoxy-$C_1$-$C_4$-Alkyl (z.B. Methoxy-methyl, Ethoxymethyl, tert.-Butoxymethyl, Methoxyethyl, Ethoxy-ethyl, Butoxy-ethyl), $C_1$-$C_4$-Alkylthio-$C_1$-$C_4$-alkyl (z.B. Methylthiomethyl, Methylthioethyl), $C_2$-$C_6$-Alkenyl (z.B. Ethenyl, Propen-1-yl, Propen-2-yl, Propen-3-yl, 2-Buten-2-yl), ggf. subst. $C_2$-$C_6$-Alkinyl (z.B.

Ethinyl, Methoxy-ethinyl, Propinyl, 3-Phenylethin-1-yl, 3-Hydroxy-propyn-1-yl, 3-Chlor-propin-1-yl), $C_1$-$C_6$-Alkoxy (z .B. Methoxy, Ethory, n-, iso-Propoxy, n-, iso-, sec.-, tert.-Butoxy), $C_1$-$C_4$-Alkylthio (z.B. Methylthio), ggf. subst. Benzylthio (z.B. 2-Chlor-benzylthio),$C_1$-$C_4$-Alkylcarbonyl (z.B. Acetyl, Ethylcarbonyl, iso-Propylcarbonyl), ggf. subst. Phenylcarbonyl (z.B. 2-Chlorphenylcarbonyl, 4-Methyl-phenylcarbonyl), ggf. subst. Aryl, Phenyl, Naphthyl (z.B. 4-Methylphenyl, 3-Hydroxy-4-Methylphenyl, 1-Naphthyl, 2-Methyl-1-naphthyl), ggf. subst. Aryloxy (z.B. Phenoxy, 2-Methyl-phenoxy, 4-Chlorphenoxy, 3-Nitrophenoxy), ggf. subst. Arylthio (z.B. Phenylthio), ggf. subst. Aryl-$C_1$-$C_4$-Alkyl (z.B. Benzyl, 2-Chlor-benzyl, 2,5-Dimethoxybenzyl, Phenethyl, 4-Methyl-phenethyl), ggf. subst, Aryl-$C_2$-$C_4$-alkenyl (Phenylethenyl, 2-Chlor-phenyl-ethenyl), ggf. subst. Aryl-$C_2$-$C_4$-alkinyl (Phenylethinyl, 4-Methyl-phenylethinyl, 2-Naphthylethinyl), ggf. subst. Aryloxy-$C_1$-$C_4$-alkyl (z.B. Phenoxymethyl, 2-CN-Phenoxymethyl, 1-Naphthyloxymethyl, Phenoxyethyl), ggf. subst. Arylthio-$C_1$-$C_4$-alkyl (z.B. Phenylthiomethyl), ggf. subst. Heteroaryl (z.B. Pyridyl, Thienyl, 2-Chlor-thien-5-yl), ggf. subst. Heteroaryloxy (z.B. 2-Pyridyloxy, 2-Benzothienyloxy, 2-Benzoxazolyloxy), ggf. subst. Heteroaryl-$C_1$-$C_4$-alkyl (z.B. 4-Pridylmethyl, 4-Chlor-2-Thienylmethyl), ggf. subst. Heteroaryl-$C_2$-$C_4$-alkenyl (z.B. 2-Pyridylethenyl, 6-Chlor-2-pyridylethenyl, 2-Thiazolylethenyl), ggf. subst. Heteroaryloxy-$C_1$-$C_4$-alkyl (z.B. 2-Pyridyloxymethyl, 2-Benzoxazolyloxymethyl, 6-Methyl-2-Benzthiazolyloxyethyl), wobei "ggf. subst." die Reste Halogen, Cyano, Nitro, $C_1$-$C_4$-Alkyl (z.B. Methyl, Ethyl), $C_1$-$C_4$-Alkoxy (z.B. Methoxy, Ethoxy, tert. -Butoxy),$C_1$-$C_4$-Halogenalkyl(z.B.Trifluormethyl),$C_1$-$C_4$-Halogenalkoxy (z.B. Trifluormethoxy,$C_1$-$C_4$-Alkoximino-$C_1$-$C_2$-alkyl (z.B. Methoximinomethyl, Ethoximinomethyl, Methoximinoethyl) bedeutet und dem Ausdruck Heteroaryl die zuvor angegebene Bedeutung zukommt und

$R^1$, $R^2$, $R^3$, $R^4$, $R^5$

unabhängig voneinander Wasserstoff oder $C_1$-$C_4$-Alkyl (z.B. Methyl, Ethyl, n-, iso-Propyl, n-, iso-, sec-, tert.-Butyl) bedeuten und

U, V, W

unabhängig voneinander Wasserstoff, Halogen, $C_1$-$C_4$-Alkyi (z.B. Methyl, Ethyl, Propyl, Butyl) oder $C_1$-$C_4$-Alkoxy (z.B. Methoxy, Ethoxy) bedeuten.

Bevorzugt werden Verbindungen I, in denen A für $=CHOCH_3$ oder $=NOCH_3$ steht.

Außerdem werden Verbindungen I bevorzugt, in denen B Methoxy oder Methylamino bedeutet.

Daneben werden Verbindungen I bevorzugt, in denen U, V und W gleichzeitig Wasserstoff bedeuten.

Des weiteren werden Verbindungen I bevorzugt, in denen Het für einen unsubstituierten oder substituierten Heterocyclus der folgenden Gruppe steht: Thiazolyl, Benzthiazolyl, Isoxazolyl, Thiadiazolyl, Oxadieazolyl und Thienyl.

Insbesondere werden Verbindungen I bevorzugt, in denen Het für einen unsubstituierten oder substituierten Heterocyclus der folgenden Gruppe steht: Thiazol-2-yl, Benzthiazol-2-yl, Isoxazol-3-yl, 1,3,4-Thiadiazol-2-yl, 1,3,4-Oxadiazol-2-yl und Thien-2-yl.

Als Substituenten von Het kommen bevorzugt die folgenden Gruppen in Betracht:

$C_1$-$C_6$-Alkyl, welches partiell oder vollständig halogeniert sein kann (d.h. in dem die Wasserstoffatome zum Teil oder vollständig durch Halogenatome aus der Gruppe Fluor, Chlor und/oder Brom ersetzt sein können), und/oder welches einen der folgenden Reste tragen kann: Hydroxy, $C_1$-$C_4$-Alkoxy, $C_1$-$C_4$-Alkylcarbonyloxy, Phenyl, Naphthyl, Phenoxy, Naphthyloxy, Benzoyloxy, Naphthoyloxy oder Thienyl, wobei die aromatischen Gruppen ihrerseits ein bis drei der folgenden Reste tragen können: Cyano, Nitro, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio,

$C_3$-$C_8$-Cycloalkyl, welches partiell halogeniert sein kann und/oder ein bis drei der folgenden Gruppen tragen kann: $C_1$-$C_4$-Alkyl, $C_1$-$C_4$-Halogenalkyl, $C_1$-$C_4$-Alkoxy und $C_1$-$C_4$-Halogenalkoxy,

$C_2$-$C_6$-Alkoxy, welches partiell oder vollständig halogeniert sein kann (d.h. in dem die Wasserstoffatome zum Teil oder vollständig durch Halogenatome aus der Gruppe Fluor, Chlor und/oder Brom ersetzt sein können), und/oder welches einen der folgenden Reste tragen kann: Phenyl, Naphthyl, Phenoxy, Naphthyloxy, Benzoyloxy, Naphthoyloxy oder Thienyl, wobei die aromatischen Gruppen ihrerseits partiell oder vollständig halogeniert sein können und/oder ein bis drei der folgenden Reste tragen können: Cyano, Nitro, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio,

Phenyl, Imidazolyl, Thienyl, Benzothienyl und Pyridyl, wobei diese Gruppen ihrerseits partiell oder vollständig halogeniert sein können und/oder ein bis drei der folgenden Reste tragen können: Cyano, Nitro, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio.

Bevorzugt werden die Verbindungen, bei denen

A

$=CHOCH_3$ oder $=NOCH_3$ bedeutet

B

$OCH_3$ oder $NHCH_3$ bedeutet,

U, V, W

Wasserstoff bedeuten und
Het, R und $R^4$,$R^5$ die vorstehend aufgeführte Bedeutung haben.

Die neuen Verbindungen der Formel I eignen sich als Fungizide.

Die neuen Verbindungen, bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewunschtenfalls unter Verwendung von Emulgiermitteln und Dispergiermitteln, wobei im Falle von Wasser als Verdunnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoryethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:

I. eine Lösung aus 90 Gew.-Teilen einer erfindungsgemäßen Verbindung und 10 Gew.-Teilen N-Methyl-$\alpha$-pyrrolidon, die zur Anwendung in Form kleinster Tropfen geeignet ist;

II. eine Mischung aus 20 Gew.-Teilen einer erfindungsgemäßen Verbindung, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.

III. eine wäßrige Dispersion aus 20 Gew.-Teilen einer erfindungsgemäßen Verbindung, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen einer erfindungsgemäßen Verbindung, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen einer erfindungsgemäßen Verbindung, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen einer erfindungsgemäßen Verbindung und 97 Gew.-Teilen feinteiligem Kaolin; dieses Stäubemittel enthält 3 Gew.-% Wirkstoff;

VII. eine innige Mischung aus 30 Gew.-Teilen einer erfindungsgemäßen Verbindung, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen einer erfindungsgemäßen Verbindung, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen einer erfindungsgemäßen Verbindung, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-harnstoff-formaldehydKondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaecualis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,

Dithiocarbamate und deren Derivate, wie Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester;
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-βbis-(dimethylamino)-phosphinyl'-3-phenyl-1,2,4-triazol,
2,3-Dicyano-1,4-dithioanthrachinon,
2-Thio-1,3-dithioloβ4,5-b'chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophtalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methy-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
α-(2-Chlorphenyl)-α-(4-chlorphenyl)-5-pyrimidin-methanol,

5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-83-methoxycarbonyl-2-thioureido)-benzol,
sowie verschiedene Fungizide, wie Dodecylguanidinacetat,
3-[3-(3,5-Dimethyl-2-oxycyclohexyl)-2-hydroxyethyl)]glutarimid, Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorhenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Synthesebeispiele

Die in den nachstehenden Synthesebeispielen wiedergegebenen Vorschriften wurden unter entsprechender Abwandlung der Ausgangsstoffe zur Gewinnung weiterer Verbindungen I benutzt. Die so erhaltenen Verbindungen sind in den nachstehenden Tabellen mit physikalischen Angaben aufgeführt.

Beispiel 1

o-Formylphenylglyoxylsäuremethylester-O-methyloxim (Verbindung A)

20 g (0,07 mol) 2-Brommethylphenylglyoxylsäuremethylester-O-methyloxim werden in 300 ml $CCl_4$ gelöst und mit 32 g (0,236 mol) N-Methylmorpholin-N-Oxi•$H_2O$ versetzt. Man erhitzt 4 Stunden unter Rückfluß. Anschließend läßt man abkühlen, wäscht mit Wasser und mit 10 %-iger Salzsäure, trocknet und engt ein. Nach Chromatographie an Kieselgel mit Cyclohexan/Essigester verbleiben 9,5 g (61 %) Aldehyd (A) als farbloser Feststoff.
$^1$H-NMR ($CDCl_3$/TMS) : $\delta$ = 3,86, 4,01 (s,3H), 7,32-7,93 (m,4H), 9,88 ppm (s,1H).

Beispiel 2

2-(Formylhydroxylamino)phenylglyoxylsäuremethylester-O-methyloxim (Verbindung B)

Zu einer Lösung von 7,15 g (67,5 mmol) $Na_2CO_3$ und 4,7 g (67,5 mmol) Hydroxylaminhydrochlorid in 50 ml Isopropanol gibt man bei 60 - 70 °C portionsweise 10 g (45 mmol) Aldehyd A. Nach beendeter Zugabe läßt man abkühlen und rührt über Nacht bei RT nach. Man engt ein, nimmt den Rückstand in Essigester auf, wäscht mit Wasser, trocknet und engt ein. Nach Chromatographie mit Cyclohexan/Essigester verbleiben 7,5 g (71 %) Oxim (B).
$^1$H-NMR ($CDCl_3$/TMS) :$\delta$ = 3,86, 4,03 (s,3H) , 7,20-7,68 (m,4H), 8,00 (s,1H), 8,34 ppm (br,1H).

Beispiel 3

2-Hydroxylcarbonylphenylglyoxylsäuremethylester-O-methyloxim (Verbindung C)

Eine Mischung aus 10 g (45 mmol) der Verbindung A, 5,5 g (55 mmol) Chromtrioxid und 100 ml konz. Essigsäure wird 2 Stunden bei 50 °C gerührt. Man läßt abkühlen, verdünnt mit Wasser, extrahiert mit Essigester, trocknet und engt ein. Es verbleiben 9,9 g (92,5 %) der Benzoesäure C als Öl.
$^1$H-NMR (DMSO-$d^6$) : $\delta$ = 3,70, 3,89 (s,3H), 7,28-7,89 (m,4H), 12,8 ppm (br,1H).

Beispiel 4

2-Chlorcarbonylphenylglyoxylsäuremethylester-O-methyloxim (Verbindung D)

Zu einer Lösung von 2 g (8,43 mmol) der Benzoesäure C und 1,3 g (16,40 mmol) Pyridin in 20 ml Methyl-tert.-butylether und 10 ml Methylenchlorid tropft man bei -10 - 0 °C 1,1 g (9,3 mmol) Thionylchlorid. Man läßt die Mischung auf Raumtemperatur erwärmen und rührt über Nacht bei Raumtemperatur ($R_T$). Anschließend wird das Lösungsmittel im Vakuum entfernt. Das Rohprodukt kann in dieser Form für die weiteren Umsetzungen eingesetzt werden.

Beispiel 5

2-Bromphenylglyoxylsäuremethylester-O-methyloxim (Verbindung E)

Zu einer Lösung von 10 g (41 mmol) 2-Brom-phenylglyoxylsäuremethylester in 30 ml Methanol gibt man 5,2 g (62 mmol) O-Methylhydroxylaminhydrochlorid und erhitzt 2 Stunden unter Rückfluß. Man engt ein, nimmt den Rückstand in Essigester auf und wäscht mit Wasser. Nach dem Trocknen und Einengen verbleiben 6,9 g (62 %) der Verbindung E.
$^1$H-NMR ($CDCl_3$/TMS) $\delta$ = 3,88, 4,07 (s,3H), 7,17-7,61 ppm (m,4H).

Beispiel 6

2-Ethinylphenylglyoxylsäure-methylester-O-methyloxim (Verbindung F)

Zu einer Lösung von 55,4 g (0,23 mol) 2-Bromphenylglyoxylsäuremethylester in 415 ml Triethylamin gibt man 33,3 g (0,35 mol) Trimethylsilylacetylen, 3,8 g Palladium(II)acetat, 3,2 g Kupfer(I)jodid und 8,9 g Triphenylphosphin und leitet anschließend 30 min. Stickstoff durch die Lösung. Das Reaktionsgemisch wird anschließend 45 min. auf 90 °C erhitzt. Man läßt abkühlen und filtriert ab. Das Filtrat wird eingeengt, in Essigester aufgenommen und mit Wasser gewaschen. Die organische Phase wird getrocknet und eingeengt. Es verbleiben 56,8 g $\alpha$-Ketoester als schwarzes Öl.

Das auf diese Weise dargestellte Rohprodukt wird in 50 ml Methanol gelöst, mit 38,9 g (0,37 mol) O-Methylhydroxylaminhydrochlorid versetzt und 15 min. auf 60 °C erhitzt. Man engt ein, nimmt den Rückstand in Essigester auf und wäscht mit Wasser. Die organische Phase wird getrocknet und eingeengt. Es verbleiben 52,4 g der Trimethylsilyl-Verbindung als schwarzes Öl.
$^1$H-NMR ($CDCl_3$/TMS) : $\delta$ = 0,22 (s,9H,$SiMe_3$); 3,86; 4,06 (s,3H,$OCH_3$); 7,25 - 7,61 ppm (m,4H,Aryl).

Die auf diese Weise dargestellte Trimethylsilylacetylen-Verbindung wird in 320 ml Methanol gelöst und zusammen mit 3,2 g Kaliumcarbonat 1 Stunde bei Raumtemperatur (20 °C) gerührt. Man engt anschließend ein, nimmt den Rückstand in Methylenchlorid auf und wäscht mit 10 %iger Natriumhydrogencarbonat-Lösung Die organische Phase wird getrocknet und eingeengt. Es verbleiben 36 g (72 % bezogen auf 2-Brom-phenylglyoxylsäuremethylester) der Verbindung F als schwarzer Feststoff.
$^1$H-NMR ($CDCl_3$/TMS) : $\delta$ = 3,17 (s,3H,≡C-H); 3,87; 4,07 (s,3H,$OCH_3$); 7,27-7,60 ppm (m,4H,Aryl).

Beispiel 7

2 (5-Phenyl-isoxazol-3-yl) phenylglyoxylsäuremethylester-O-methyloxim (Verbindung 37, Tab. I)

700 mg (7,6 mmol) Phenylacetylen und 1,2 g (5 mmol) der Verbindung B werden in 20 ml Methylenchlorid vorgelegt. Man gibt 5,7 g (7,6 mmol) Natriumhypochlorid und 3 Tropfen verdünnte NaOH zu und rührt über Nacht bei Raumtemperatur. Die Methylenchloridphase wird abgetrennt und eingeengt. Nach Chromatographie an Kieselgel mit Cyclohexan/Essigester erhält man 700 mg (42 %) der Verbindung 37, Tab. I als Öl.
$^1$H-NMR ($CDCl_3$/TMS) : $\delta$ = 3,80, 4,01 (s,3H) , 6,67 (s,2H), 7,35-7,80 ppm (m,9H).

Beispiel 8

2[3-(2-Methylphenyl)-isoxazol-5-yl]-phenylglyoxylsäuremethylester-O-methyloxim (Verbindung 38, Tab. I)

1 g (3,15 mmol) Verbindung F und 600 mg (4,73 mmol) 2-Methylbenzaldehyd-oxim werden in 50 ml Methylenchlorid vorgelegt. Man gibt 4,5 g Natriumhypochlorid zu und rührt 1 Stunde bei Raumtemperatur. Die organische Phase wird abgetrennt, getrocknet und eingeengt. Nach Chromatographie an Kieselgel erhält man 1 g der Verbindung 38, Tab. I als Öl.
$^1$H-NMR ($CDCl_3$/TMS) : $\delta$ = 2,48, 3,79, 4,02 (s,3H), 6,52(s,1H), 7,26-7,94 ppm (8H).

Beispiel 9

2[5-(3-Methylphenyl)-1,3,4-oxadiazol-2-yl]-phenylglyoxylsäuremethylester-O-methyloxim (Verbindung 50, Tab. I)

a) Zu einer Lösung von 3 g (13,6 mmol) der Verbindung A in 100 ml Methanol gibt man 2 g (13,6 mmol) 3-Methylphenylhydrazon und rührt über Nacht bei Raumtemperatur. Der ausgefallene Feststoff wird abgesaugt, mit Diisopropylether gewaschen und im Vakuum getrocknet. Es verbleiben 3,6 g (75 %) des Iminohydrazons als Feststoff. Schmp.: 231 - 232 °C.
b) Zu einer Lösung von 1,6 g (4,5 mmol) der unter a) hergestellten Iminohydrazon-Verbindung in 100 ml Chloroform gibt man 4,2 g (9,5 mmol) Bleitetraacetat und rührt über Nacht bei Raumtemperatur. Man gibt 750 ml Wasser zu, trennt die organische Phase ab, trocknet und engt ein. Es verbleiben 1,5 g (95 %) der Verbindung 50, Tab. I als Feststoff. Schmp.: 166 - 167 °C.

Beispiel 10

2(Thiazol-2-yl)phenylglyoxylsäuremethylester-O-methyloxim (Verbindung 1, Tab. I)

Unter Stickstoff gibt man zu einer Mischung aus 1,4 g (5,19 mmol) der Brom-Verbindung (Verbindung E), 100 mg Palladium(II)chlorid und 310 mg Triphenylphosphin in 30 ml Tetrahydrofuran 1,7 g 2-Triethylstannyl-thiazol und erhitzt 2 Stunden unter Rückfluß. Anschließend wird filtriert und eingeengt. Nach Chromatographie an Kieselgel mit Cyclohexan/Essigester verbleiben 700 mg (49 %) der Verbindung 1, Tab. I als Öl.
$^1$H-NMR ($CDCl_3$/TMS) $\delta$ = 3,75, 3,97 (s,3H), 7,28 - 7,86 ppm (m,6H).

Beispiel 11

2[5-Cyclohexyl-1,3,4-thiadiazol-2-yl]phenylglyoxylsäuremethylester-O-methyloxim (Verbindung 48, Tab. I)

Bei 0 °C gibt man zu einer Lösung von 2,1 g Hydrazinhydrat in 7 ml Wasser 3,6 g Dithiocyclohexancarbonsäuremethylester in 25 ml Methanol zu. Man läßt 10 min. nachrühren und gießt dann auf 100 ml Wasser. Anschließend wird mit 10 %iger Salzsäure neutralisiert und mit Methyl-tert.-butylether extrahiert, getrocknet und eingeengt. Der Rückstand wird in 50 ml Acetonitril aufgenommen. Bei 0 °C tropft man eine Lösung von 5,5 g (23 mmol) der Verbindung D in 30 ml Acetonitril zu. Anschließend wird 2 Stunden unter Rückfluß erhitzt. Anschließend gibt man Methylenchlorid zu, wäscht mit ges. $NaHCO_3$-Lösung und mit Wasser, trocknet und engt ein.

Nach Chromatographie an Kieselgel mit Cyclohexan/Essigester verbleiben 3,5 g (49 %) der Verbindung 48, Tab. I als Öl.
$^1$H-NMR ($CDCl_3$/TMS) $\delta$ = 1,26 - 3,21 (m,11H), 3,81, 3,96 (s,3H) 7,30 -7,81 ppm (m, 4H).

Beispiel 12

2[Benzthiazol-2-yl]-phenylglyoxylsäuremethylester-O-methyloxim (Verbindung 2, Tab. I)

a) Unter Stickstoff tropft man bei -78 °C 1,5 Eq n-BuLi zu einer Lösung von 2 g (6,9 mmol) 2-Benzthiazol-2-yl-brombenzol in 20 ml THF. Man rührt 1 Stunde bei dieser Temperatur und gibt dann 1,6 g Oxalsäure-dimethylester in 20 ml THF zu. Man läßt die Mischung sich erwärmen, gibt Wasser zu und extrahiert mit Methyl-tert.-butylether. Nach Chromatographie an Kieselgel mit Hexan/Methyl-tert.-butylether verbleiben 300 mg (15 %) des entsprechenden Phenylketoesters.
$^1H$ = NMR ($CDCl_3$/TMS) : $\delta$ = 3,56 (s,3H), 7,38 - 8,01 ppm (m,8H).
b) Zu einer Lösung des so dargestellten Ketoesters in 5 ml Methanol gibt man 1 g Methoxyaminhydrochlorid. Nach 1 scündigem Rühren bei Raumtemperatur wird eingeengt. Chromatographie an Kieselgel mit Hexan/Methyl-tert.-butylether liefert 100 mg der Verbindung 2, Tab. I als grauen Feststoff.
$^1H$-NMR ($CDCl_3$/TMS) : $\delta$ = 3,76, 3,99 (s,3H), 7,35 - 8,03 ppm (m,8H).

Tabelle I

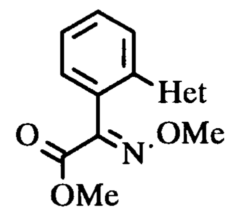

Ia

| Nr. | Het | physik. Daten; H-NMR [δ ,ppm], IR[$cm^{-1}$]; m.p. [°C] |
|---|---|---|
| 1 | Thiazol-2-yl | 3,75; 3,97 (s,3H); 7,28-7,86 (m,6H) |
| 2 | Benzthiazol-2-yl | 3,76; 3,99 (s,3H); 7,35-8,03 (m,8H) |
| 3 | Thien-2-yl | |
| 4 | Benzthien-2-yl | |
| 5 | Furan-2-yl | |
| 6 | Furan-3-yl | |
| 7 | Benzofuran-2-yl | |
| 8 | Benzofuran-3-yl | |
| 9 | Imidazol-2-yl | |
| 10 | Benzimidazol-2-yl | |
| 11 | Pyrrol-2-yl | |
| 12 | N-Methyl-pyrrol-2-yl | |

| Nr. | Het | physik. Daten; H-NMR [δ ,ppm], IR[$cm^{-1}$]; m.p. [°C] |
|---|---|---|
| 13 | Pyridin-2-yl | |
| 14 | Pyridin-3-yl | |
| 15 | Pyridin-4-yl | |
| 16 | Chinolin-2-yl | |
| 17 | 6-Chlor-pyridin-2-yl | |
| 18 | 4-Methyl-pyridin-2-yl | |
| 19 | Oxazol-2-yl | |
| 20 | Benzoxazol-2-yl | |
| 21 | 4-Phenyl-oxazol-2-yl | |
| 22 | 5-Phenyl-oxazol-2-yl | |
| 23 | 5-Methyl-isoxazol-3-yl | |
| 24 | 5-Brommethyl-isoxazol-3-yl | 3,81; 3,99 (s,3H); 4,48 (s,2H); 6,45; (s,1H); 7,32-7,71 (m,4H) |
| 25 | 5-Hydroxymethyl-isoxazol-3-yl | 3490, 1717, 1370, 1255, 1081, 1068, 1023, 953, 778 |
| 26 | 5-Acyloxymethyl-isoxazol-3-yl | 2,13; 3,81; 3,99(s,3H); 5,18 (s,2H); 6,47(s,1H); 7,32-7,71 (m,4H) |
| 27 | 5-(2-Methyl)phenyloxymethyl-isoxazol-3-yl | 2,25; 3,77; 3,95 (s,3H); 5,14 (s,2H); 6,47 (s,1H); 6,84-7,70 (m,8H) |
| 28 | 5-(2,5-Dimethyl)phenyloxymethyl-isoxazol-3-yl | 2,20; 2,32; 3,77; 3,96 (s,3H); 5,14 (s,2H); 6,47 (s,1H); 6,67-7,71 (m,7H) |

| Nr. | Het | physik. Daten; H-NMR [δ ,ppm], IR[$cm^{-1}$]; m.p. [°C] |
|---|---|---|
| 29 | 5-tert.-Butyl-isoxazol-3-yl | 1,36 (s,9H); 3,77; 3,96 (s,3H); 6,09 (s,1H); 7,30-7,70 (m,4H) |
| 30 | 5-Cyclohexyl-isoxazol-3-yl | 1,13-2,85 (m,11H); 3,78; 3,99 (s,3H); 6,11 (s,1H); 7,30-7,71 (m,4H) |
| 31 | 5-Pentyl-isoxazol-3-yl | 0,71-2,75 (m,11H); 3,79; 4,00 (s,3H); 6,11 (s,1H); 7,30-7,71 (m,4H) |
| 32 | 5-[1-(4-Nitrophenoxy)ethyl]-isoxazol-3-yl | 1,80 (d,3H); 3,76; 3,94 (s,3H); 5,60 (q,1H); 6,37 (s,1H); 6,97-8,21 (m,8H) |
| 33 | 5-Benzoyloxymethyl-isoxazol-3-yl | 3,79; 3,97 (s,3H); 5,43 (s,2H); 6,54 (s,1H); 7,28-8,08 (m,9H) |
| 34 | 5-ß-Naphthyloxymethylisoxazol-3-yl | 3,74; 3,92 (s,3H); 5,25 (s,2H); 6,53 (s,1H); 7,18-7,78 (m,11H) |
| 35 | 5-α-Naphthyloxymethylisoxazol-3-yl | 3,77; 3,96 (s,3H); 5,35 (s,2H); 6,57 (s,1H); 6,85-8,28 (m,11H) |
| 36 | 5-Thiophenylmethylisoxazol-3-yl | 3,75; 3,93 (s,3H); 4,13 (s,2H); 6,22 (s,1H); 7,20-7,63 (m,9H) |
| 37 | 5-Phenyl-isoxazol-3-yl | 3,80; 4,01 (s,3H); 6,67 (s,1H); 7,35-7,80 (m,9H) |
| 38 | 3-(2-Methyl)phenyl-isoxazol-5-yl | 2,48; 3,79; 4,02 (s,3H); 6,52 (s,1H); 7,26-7,94 (m,8H) |
| 39 | 3-Phenyl-isoxazol-5-yl | 3,79; 4,02 (s,3H); 6,65 (s,2H); 7,32-7,88 (m,9H) |
| 40 | 5-(2-Methyl)phenyl-isoxazol-3-yl | 2,52; 3,79; 4,01 (s,3H), 6,55 (s,1H); 7,28-7,70 (m,8H) |

| Nr. | Het | physik. Daten; H-NMR [δ ,ppm], IR[$cm^{-1}$]; m.p. [°C] |
|---|---|---|
| 41 | 5-(1-tert.-Butoxy)ethylisoxazol-3-yl | 1,22 (s,9H); 1,47 (d,3H); 3,77; 3,96 (s,3H); 4,78 (q,1H); 6,31 (s,1H); 7,30-7,70 (m,4H) |
| 42 | 5-n-Propyl-isoxazol-3-yl | 0,99 (t,3H); 1,74 (m,2H); 2,73 (t,2H); 3,77; 3,97 (s,3H); 6,10 (s,1H); 7,28-7,68 (m,4H) |
| 43 | 5-(2-Acetyloxy)isopropylisoxazol-3-yl | 1,80 (s,6H); 2,04; 3,79; 3,97 (s,3H); 6,29 (s,1H); 7,28-7,70 (m,4H) |
| 44 | 5-Cyclooctyl-isoxazol-3-yl | 1,22-2,12 (m,15H); 3,77; 3,97 (s,3H); 6,31 (s,1H); 7,30-7,70 (m,4H) |
| 45 | 3-tert.-Butyl-isoxazol-5-yl | 1,34 (s,9H); 3,76; 4,01 (s,3H); 6,26 (s,1H); 7,30-7,84 (m,4H) |
| 46 | 3-Cyclohexyl-isoxazol-3-yl 5 | 1,26-2,81 (m,11H); 3,76; 4,01 (s,3H); 6,20 (s,1H); 7,30-7,83 (m,4H) |
| 47 | 5-α-Methoxybenzyl-isoxazol-3-yl | 3,43; 3,71; 3,91 (s,3H); 5,36; 6,30 (s,1H); 7,28-7,66 (m,9H) |
| 48 | 5-Cyclohexyl-1,3,4-thiadiazol-2-yl | 1,26-3,21 (m,11H); 3,81; 3,96 (s,3H); 7,30-7,81 (m,4H) |
| 49 | 5-Phenyl-1,3,4-oxadiazol-2-yl | 160 - 161°C |
| 50 | 5-(3-Methyl)phenyl-1,3,4-oxadiazol-2-yl | 166 - 167°C |
| 51 | 5-(4-Cyano)phenyl-1,3,4-oxadiazol-2-yl | 188 - 189°C |
| 52 | 5-(4-Chlor)phenyl-1,3,4-oxadiazol-2-yl | 161 - 162°C |
| 53 | 5-(3,4,5-Trimethoxy)phenyl-1,3,4-oxadiazol-2-yl | 136 - 144°C |

| Nr. | Het | physik. Daten; H-NMR [$\delta$ ,ppm], IR[$cm^{-1}$]; m.p. [°C] |
|---|---|---|
| 54 | 5-(5-Methyl)imidazol-4-yl-1,3,4-oxadiazol-2-yl | 198°C (Zers.) |
| 55 | 5-Thien-2-yl-1,3,4-oxadiazol-2-yl | 196 - 197°C |
| 56 | 5-Pyridin-3-yl-1,3,4-oxadiazol-2-yl | 168 - 170°C |
| 57 | 5-Benzothien-2-yl-1,3,4-oxadiazol-2-yl | 209 - 213°C |
| 58 | 5-(3-Chlor)benzothien-2-yl-1,3,4-oxadiazol-2-yl | 205 - 206°C |
| 59 | 5-Furan-2-yl-1,3,4-oxadiazol-2-yl | 162 - 170°C |
| 60 | 5-[(3-Methoxy-5-thien-2-yl)phenyl] thien-2-yl | 3,68; 3,88; 4,04 (s,3H); 6,92- 7,58 (m,12H) |

Tabelle II

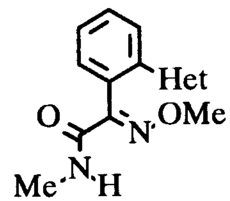

Ih

| Nr. | Het | physik. Daten |
|---|---|---|
| 1 | Thiazol-2-yl | |
| 2 | Benzthiazol-2-yl | |
| 3 | Thien-2-yl | |
| 4 | Benzthien-2-yl | |
| 5 | Furan-2-yl | |
| 6 | Furan-3-yl | |
| 7 | Benzofuran-2-yl | |
| 8 | Benzofuran-3-yl | |
| 9 | Imidazol-2-yl | |
| 10 | Benzimidazol-2-yl | |
| 11 | Pyrrol-2-yl | |
| 12 | N-Methyl-pyrrol-2-yl | |
| 13 | Pyridin-2-yl | |

| Nr. | Het | physik. Daten |
|---|---|---|
| 14 | Pyridin-3-yl | |
| 15 | Pyridin-4-yl | |
| 16 | Chinolin-2-yl | |
| 17 | 6-Chlor-pyridin-2-yl | |
| 18 | 4-Methyl-pyridin-2-yl | |
| 19 | Oxazol-2-yl | |
| 20 | Benzoxazol-2-yl | |
| 21 | 4-Phenyl-oxazol-2-yl | |
| 22 | 5-Phenyl-oxazol-2-yl | |
| 23 | 5-Methyl-isoxazol-3-yl | |
| 24 | 5-Brommethyl-isoxazol-3-yl | |
| 25 | 5-Hydroxymethyl-isoxazol-3-yl | |
| 26 | 5-Acyloxymethyl-isoxazol-3-yl | |
| 27 | 5-(2-Methyl)phenyloxymethyl-isoxazol-3-yl | |
| 28 | 5-(2,5-Dimethyl)phenyloxy-methyl-isoxazol-3-yl | |
| 29 | 5-tert.-Butyl-isoxazol-3-yl | |
| 30 | 5-Cyclohexyl-isoxazol-3-yl | |
| 31 | 5-Pentyl-isoxazol-3-yl | |
| 32 | 5-[1-(4-Nitrophenoxy)ethyl]-isoxazol-3-yl | |
| 33 | 5-Benzoyloxymethyl-isoxazol-3-yl | |
| 34 | 5-ß-Naphthyloxymethyl-isoxazol-3-yl | |

| Nr. | Het | physik. Daten |
|---|---|---|
| 35 | 5-α-Naphthyloxymethyl-isoxazol-3-yl | |
| 36 | 5-Thiophenylmethyl-isoxazol-3-yl | |
| 37 | 5-Phenyl-isoxazol-3-yl | |
| 38 | 3-(2-Methyl)phenyl-isoxazol-5-yl | |
| 39 | 3-Phenyl-isoxazol-5-yl | |
| 40 | 5-(2-Methyl)phenyl-isoxazol-3-yl | |
| 41 | 5-(1-tert.-Butoxy)ethyl-isoxazol-3-yl | |
| 42 | 5-n-Propyl-isoxazol-3-yl | |
| 43 | 5-(2-Acetyloxy)isopropyl-isoxazol-3-yl | |
| 44 | 5-Cyclooctyl-isoxazol-3-yl | |
| 45 | 3-tert.-Butyl-isoxazol-5-yl | |
| 46 | 3-Cyclohexyl-isoxazol-5-yl | |
| 47 | 5-α-Methoxybenzyl-isoxazol-3-yl | |
| 48 | 5-Cyclohexyl-1,3,4-thiadiazol-2-yl | |
| 49 | 5-Phenyl-1,3,4-oxadiazol-2-yl | |
| 50 | 5-(3-Methyl)phenyl-1,3,4-oxadiazol-2-yl | |
| 51 | 5-(4-Cyano)phenyl-1,3,4-oxadiazol-2-yl | |
| 52 | 5-(4-Chlor)phenyl-1,3,4-oxadiazol-2-yl | |
| 53 | 5-(3,4,5-Trimethoxy)phenyl-1,3,4-oxadiazol-2-yl | |
| 54 | 5-(5-Methyl)imidazol-4-yl-1,3,4-oxadiazol-2-yl | |
| 55 | 5-Thien-2-yl-1,3,4-oxadiazol-2-yl | |

| Nr. | Het | physik. Daten |
|---|---|---|
| 56 | 5-Pyridin-3-yl-1,3,4-oxadiazol-2-yl | |
| 57 | 5-Benzothien-2-yl-1,3,4-oxadiazol-2-yl | |
| 58 | 5-(3-Chlor)benzothien-2-yl-1,3,4-oxadiazol-2-yl | |
| 59 | 5-Furan-2-yl-1,3,4-oxadiazol-2-yl | |
| 60 | 5-[(3-Methoxy-5-thien-2-yl)phenyl]thien-2-yl | |

Anwendungsbeispiele

Für die folgenden Anwendungsbeispiele wurden als Vergleichsverbindungen die Verbindungen 2-(2-Phenylphenyl)-glyoxysäuremethylester-O-methyloxium (A) - entspricht EP-A 254 426 - und 2-(2-Phenylphenyl)-3-methoxy-acrylsäuremethylester (B) - bekannt aus EP-A 178 826 - verwendet.

Anwendungsbeispiel 1

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

Das Ergebnis des Versuchs zeigt, daß die Verbindungen aus Tabelle 1, Nr. 21, 32, 37, 38, 39, 40 und 45 bei der Anwendung als 63 ppm Wirkstoff enthaltende Spritzbrühe eine bessere fungizide Wirkung zeigen (95 %) als die bekannten Vergleichswirkstoffe A und B (70 %).

Anwendungsbeispiel 2

Wirksamkeit gegen Pyricularia oryzae (protektiv)

Blätter von in Töpfen gewachsenen Reiskeimlingen der Sorte "TaiNong" wurden mit wäßriger Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24°C und 95 bis 99 % relativer Luftfeuchtigkeit aufgestellt. Nach 6 Tagen wurde das Ausmaß des Krankheitsbefalls ermittelt.

Das Ergebnis des Versuchs zeigt, daß die Verbindungen aus Tabelle 1, Nr. 29, 40, 41, 42, 43, 44, 45, 46 und 50 bei der Anwendung als 250 ppm Wirkstoff enthaltende Spritzbrühe eine bessere fungizide Wirkung zeigen (85 %) als der bekannte Vergleichswirkstoff A (20 %).

**Patentansprüche**

**1.** Diarylderivate der allgemeinen Formel I

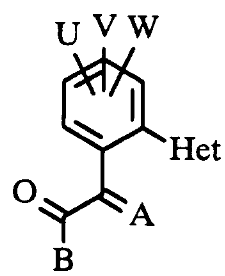

I

in der die Substituenten die folgende Bedeutung haben:

A

$=CHOR^1$, $=CHSR^1$, $=CHR^1$, $=CHCl$ oder $=NOR^1$,

B

$OR^2$, $SR^2$ oder $NR^2R^3$,

Het

ein ein-, zwei- oder dreikerniger aromatischer Fünfring- oder Sechsringheterocyclus, der gegebenenfalls einen bis drei der Reste R tragen kann,

R

unabhängig voneinander Halogen, Nitro, Cyano, $CO_2R^4$, $NR^4R^5$, $CONR^4R^5$, $S(O)_nR^4$ $P(O)(OR^4)_2$, ggf. subst. $C_1$-$C_6$-Alkyl, $C_1$-$C_4$-Halogenalkyl, ggf. subst. $C_3$-$C_6$-Cycloalkyl, $C_1$-$C_4$-Alkoxy-$C_1$-$C_4$-alkyl, $C_1$-$C_4$-

Alkylthio-$C_1$-$C_4$-alkyl, ggf. subst. $C_2$-$C_6$-Alkenyl, ggf. subst. $C_2$-$C_6$-Alkinyl, $C_1$-$C_6$-Alkoxy, $C_1$-$C_4$-Thioalkoxy, ggf. subst. Benzylthio,
$C_1$-$C_4$-Alkylcarbonyl, ggf. subst. Phenylcarbonyl, ggf. subst. Aryl, ggf. subst. Aryloxy, ggf. subst. Arylthio, ggf. subst. Aryl-$C_1$-$C_4$-alkyl, ggf. subst. Aryl-$C_2$-$C_4$-alkenyl, ggf. subst. Aryl-$C_2$-$C_4$-alkinyl, ggf. subst. Aryloxy-$C_1$-$C_4$-alkyl, ggf. subst. Arylthio-$C_1$-$C_4$-alkyl, ggf. subst. Heteroaryl, ggf. subst. Heteroaryloxy, ggf. subst. Heteroaryl-$C_1$-$C_4$-alkyl, ggf. subst. Heteroaryl-$C_2$-$C_4$-alkenyl, ggf. subst. Heteroaryloxy-$C_1$-$C_4$-alkyl bedeuten,
wobei "ggf. subst." für die Reste Halogen, Cyano, Nitro, $C_1$-$C_4$-Alkyl, $C_1$-$C_4$-Alkoxy, $C_1$-$C_4$-Halogenalkyl, $C_1$-$C_4$-Halogenalkoxy, $C_1$-$C_4$-Alkoximino-$C_1$-$C_2$-alkyl steht und
wobei
n für 0, 1 oder 2 steht,
$R^1$, $R^2$, $R^3$, $R^4$, $R^5$
unabhängig voneinander Wasserstoff oder $C_1$-$C_4$-Alkyl und
U, V, W
unabhängig voneinander Halogen, $C_1$-$C_4$-Alkyl oder $C_1$-$C_4$-Alkoxy.

**2.** Fungizid, enthaltend einen festen flüssigen Trägerstoff und eine fungizid wirksame Menge eines Diarylderivats der allgemeinen Formel I gemäß Anspruch 1.

**3.** Verfahren zur Bekämpfung von Pilzen, dadurch gekennzeichnet, daß man die Pilze oder die von Pilzbefall bedrohten Pflanzen, Saatgüter, Materialien oder den Boden behandelt mit einer fungizid wirksamen Menge eines Diarylderivats der allgemeinen Formel I gemäß Anspruch 1.

**4.** Verbindung der Formel I gemäß Anspruch 1, in der A $NOCH_3$, B $OCH_3$, Het Isoxazolyl-3, R 5-Phenyl und U, V, W Wasserstoff bedeuten.

**5.** Verbindung der Formel I gemäß Anspruch 1, in der A $NOCH_3$, B $OCH_3$, Het Isoxazolyl-5, R 3-(2-Methylphenyl) und U, V, W Wasserstoff bedeuten.

**6.** Verbindung der Formel I gemäß Anspruch 1, in der A $NOCH_3$, B $OCH_3$, Het 1,3,4-Oxadiazolyl-2, R 5-(3-Methylphenyl) und U, V, W Wasserstoff bedeuten.

**7.** Verbindung der Formel I gemäß Anspruch 1, in der A $NOCH_3$, B $OCH_3$, Het Thiazolyl-2 und U, V, W Wasserstoff bedeuten.

**8.** Verbindung der Formel I gemäß Anspruch 1, in der A $NOCH_3$, B $OCH_3$, Het 1,3,4-Thiadazolyl-2, R 5-Cyclohexyl und U, V, W Wasserstoff bedeuten.

**9.** Verbindung der Formel I gemäß Anspruch 1, in der A $NOCH_3$, B $OCH_3$, Het Benzthiazolyl-2 und U, V, W Wasserstoff bedeuten.

**10.** Verfahren zur Bekämpfung von Schädlingen, dadurch gekennzeichnet, daß man eine gegen Schädlinge wirksame Menge einer Diarylverbindung der Formel I gemäß Anspruch 1 auf Insekten, Nematoden oder Spinnmilben oder auf ihre Lebensräume einwirken läßt.